# FASCICULE DE BREVET EUROPEEN

(11) **EP 3 764 923 B1**
(45) Date de publication et mention de la délivrance du brevet: **06.04.2022**
(21) Numéro de dépôt: 19709070.7
(22) Date de dépôt: 13.03.2019
(51) Int. Cl.: A61B 17/17, A61N 7/00, A61B 90/10, A61B 34/20

(54) **KIT CHIRURGICAL A UTILISER LORS D'UNE PROCEDURE DE CRANIECTOMIE**
CHIRURGISCHES KIT ZUR VERWENDUNG IN EINER KRANIEKTOMIE
SURGICAL KIT FOR USE IN A CRANIECTOMY PROCEDURE

(30) Priorité: 14.03.2018 FR 1852178
(43) Date de publication de la demande: 20.01.2021
(73) Titulaire: ASSISTANCE PUBLIQUE, HOPITAUX DE PARIS, 75004 Paris (FR); SORBONNE UNIVERSITE, 75006 Paris (FR); Carthera, 75013 Paris (FR)
(72) Inventeur: CARPENTIER, Alexandre, 75116 PARIS (FR); VIGNOT, Alexandre, 69003 LYON (FR); CHOLVY, Matthieu, 35800 DINARD (FR)
(74) Mandataire: Be IP
(86) Numéro de dépôt international: PCT/EP2019/056246
(87) Numéro de publication internationale: WO 2019/175214

(56) Documents cités:
- WO-A1-2011/101039
- CH-A2- 711 264
- US-A1- 2014 277 019

## Description

### DOMAINE DE L'INVENTION

La présente invention concerne le domaine technique général des procédés de traitement d'un tissu cérébral - humain ou animal - par ultrasons afin d'aider un praticien dans le traitement d'une pathologie.

Plus précisément, la présente invention concerne un accessoire chirurgical utile au praticien lors de l'implantation d'un dispositif médical dans l'os crânien d'un patient.

### ARRIERE PLAN DE L'INVENTION

On connaît différentes techniques permettant de traiter un tissu cérébral.

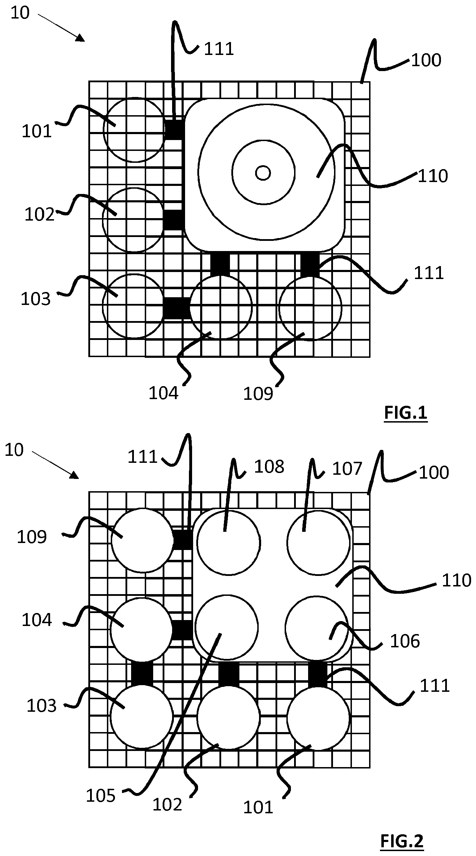

Notamment, une technique connue consiste à utiliser un dispositif intracrânien tel qu'illustré à la figure 1. Ce dispositif intracrânien 10 comprend :
- une structure support 100,
- une pluralité de transducteurs 101-109 fixés sur la structure support 100 pour la génération d'ondes ultrasonores de traitement d'une affection cérébrale,
- une borne de connexion électrique 110 montée sur la structure support 100 pour le raccordement de l'implant intracrânien à une source externe d'alimentation en énergie électrique.

Préalablement au traitement du patient, il est nécessaire d'implanter le dispositif intracrânien dans le crâne du patient. Pour ce faire, le praticien réalise une craniectomie. Une incision est faite dans le cuir chevelu, puis la peau (et les muscles le cas échéant) est soulevée (sont soulevés) afin d'exposer le crâne. Des trous de trépan sont réalisés dans le crâne aux endroits appropriés, et une scie chirurgicale (craniotome) est utilisée pour découper un volet osseux en cheminant d'un trou de trépan à un autre. On obtient ainsi un volet osseux crânien enlevable laissant place à une ouverture crânienne dans laquelle le dispositif intracrânien pourra être positionné. Le dispositif intracrânien est fixé par des moyens de fixation appropriés sur l'os à la périphérie de l'ouverture, puis le cuir chevelu et les muscles sont remis en place pour recouvrir le dispositif intracrânien.

Pour que l'ouverture crânienne soit minimale, et que la dure-mère soit protégée par le dispositif, il est souhaitable que les dimensions de l'ouverture correspondent aux dimensions minimales nécessaires pour l'ajustement du dispositif intracrânien.

Ainsi il est nécessaire de tracer sur le crâne du patient les contours de l'ouverture à réaliser préalablement à la réalisation des découpes permettant le retrait du volet osseux.

Par ailleurs, pour une bonne efficacité du traitement thérapeutique, le dispositif intracrânien - et donc l'ouverture réalisée dans le crâne du patient - doit être positionné précisément par rapport au tissu cérébral à traiter. L'utilisation des appareils de neuro-navigation per opératoire permet d'optimiser le positionnement de l'ouverture osseuse par rapport à la localisation de la lésion sous-jacente à traiter.

Le document WO 2011/101039 décrit un appareil de traitement d'affections du cerveau comportant :
- un générateur implantable incluant : un boîtier, un dispositif de traitement positionné dans ledit boîtier, et des
- des moyens de fixation du générateur implantable,
- un régulateur d'alimentation servant à alimenter en électricité le dispositif de traitement du générateur implantable.

Le document CH 711 264 décrit un dispositif implantable comportant une pluralité d'émetteurs d'ultrasons dont les ondes ultrasonores sont focalisées sur un foyer commun situé dans une région crânienne à traiter.

Le document US 2014/0277019 décrit un accessoire chirurgical destiné à être utilisé pour implanter un dispositif médical dans un os crânien d'un patient.

Un but de la présente invention est de proposer un kit chirurgical permettant d'assurer un dimensionnement optimal et un positionnement correct d'une ouverture dans un os crânien d'un patient, en particulier pour l'implantation d'un dispositif intra osseux.

### BREVE DESCRIPTION DE L'INVENTION

A cet effet, l'invention propose un kit chirurgical à utiliser lors d'une procédure de craniectomie, le kit chirurgical comprenant :
- un dispositif médical à implanter dans un os crânien d'un patient, le dispositif médical incluant une structure support et plusieurs transducteurs montés sur la structure support pour l'émission d'ondes ultrasonores de traitement d'un tissu cérébral,
- un accessoire chirurgical destiné à être positionné sur l'os crânien du patient, l'accessoire chirurgical incluant un corps dimensionné pour se rapprocher des dimensions de la structure support du dispositif médical, le périmètre externe du corps définissant les dimensions d'une ouverture à réaliser dans l'os crânien du patient pour recevoir le dispositif médical,
***remarquable en ce que*** l'accessoire chirurgical comprend une pluralité de trous ménagés dans le corps, la position de chaque trou sur le corps coïncidant avec la position de l'axe de symétrie d'un transducteur respectif sur la structure support (chaque trou définissant un repère pour l'extrémité d'un système de neuronavigation afin de faciliter le positionnement optimal (notamment par rapport à des caractéristiques de la lésion à traiter telles que sa forme et/ou sa position) de l'ouverture à réaliser dans l'os crânien).

Des aspects préférés mais non limitatifs de la présente invention sont les suivants :
- le corps peut avantageusement comprendre une lumière centrale, l'accessoire chirurgical comprenant au moins une paire de pattes opposées, notamment deux paires de pattes opposées, chaque patte s'étendant en saillie du corps vers l'intérieur de la lumière centrale ;
- la lumière centrale peut être en forme de X ;
- le corps peut présenter une forme sensiblement parallélépipédique à coins arrondis, par exemple carrée à coins arrondis, l'arrondi de chaque coin présentant un rayon de courbure sensiblement égal à la moitié du diamètre d'un foret médical de perçage (craniotome) utilisé durant la réalisation de l'ouverture dans l'os crânien du patient ;
- le corps peut présenter une forme sensiblement parallélépipédique, par exemple carrée, l'accessoire chirurgical comprenant quatre encoches en forme d'arc de cercle disposées en regard d'un coin respectif du corps ;
- la concavité de chaque encoche peut être orientée vers son coin associé, le rayon de courbure de chaque encoche étant sensiblement égal à la moitié du diamètre d'un foret médical de perçage utilisé durant la réalisation de l'ouverture dans l'os crânien du patient ;
- le corps peut être réalisé dans un matériau déformable ou semi rigide ;
- le corps peut être réalisé en silicone de qualité médicale ;
- la face inférieure du corps destinée à venir en contact avec l'os crânien du patient et/ou la face supérieure opposée à la face inférieure peuvent être texturée ;
- le kit chirurgical peut comprendre en outre au moins une entretoise apte à être positionnée entre le dispositif médical et l'os crânien du patient ;
- chaque entretoise peut comprendre un cadre périphérique incluant des orifices traversants pour le passage de vis de fixation du dispositif médical sur l'os crânien du patient ;
- chaque entretoise peut comprendre en outre des ergots s'étendant en sailli vers l'extérieur du cadre périphérique.

### BREVE DESCRIPTION DES DESSINS

D'autres avantages et caractéristiques du kit chirurgical ressortiront mieux de la description qui va suivre de plusieurs variantes d'exécution, données à titre d'exemples non limitatifs, à partir des dessins annexés sur lesquels :
- La figure 1 est une représentation schématique en vue de dessus d'un dispositif intracrânien,
- La figure 2 est une représentation schématique en vue de dessous du dispositif intracrânien,
- La figure 3 est une représentation en perspective d'une entretoise,
- La figure 4 est une représentation en perspective d'un accessoire chirurgical,
- Les figures 5 et 6 sont des représentations schématiques en vue de dessus de l'accessoire chirurgical.

### DESCRIPTION DETAILLEE DE L'INVENTION

On va maintenant décrire plus en détails un exemple de kit chirurgical selon l'invention en référence aux figures. Dans ces différentes figures, les éléments équivalents sont désignés par la même référence numérique.

Le kit chirurgical comprend un dispositif médical 10 à implanter dans un os crânien d'un patient, et un accessoire chirurgical 20 destiné à être positionné sur l'os crânien du patient.

L'accessoire chirurgical 20 permet de faciliter le positionnement d'une ouverture dans l'os crânien du patient et d'en délimiter les contours en vue de l'implantation du dispositif médical 10. Même si les formes de l'accessoire chirurgical 20 et du dispositif médical 10 ne sont pas identiques, la forme de l'accessoire chirurgical 20 dépend de la forme du dispositif médical 10. Il en va de même pour ses dimensions. En particulier, le périmètre externe de l'accessoire chirurgical 20 définit la forme et les dimensions de l'ouverture dans laquelle le dispositif médical 10 est destiné à être inséré.

Dans la suite, on décrira une variante de réalisation de l'invention en référence à un kit chirurgical dans lequel le dispositif médical 10 et l'accessoire chirurgical 20 présentent une forme sensiblement carrée, étant entendu pour l'homme du métier que les éléments de ce kit peuvent présenter d'autres formes (rectangulaire, triangulaire, parallélépipédique quelconque, etc.).

### 1. Dispositif médical

En référence aux figures 1 et 2, le dispositif médical 10 comprend :
- une structure support 100,
- des transducteurs 101-109 fixés sur la structure support 100 pour la génération d'ondes ultrasonores de traitement d'un tissu cérébral,
- une borne de connexion électrique 110 montée sur la structure support 100 pour le raccordement du dispositif médical à une unité de commande externe.

La structure support 100 peut consister en une grille réalisée en titane. Elle présente une forme carrée et peut s'étendre sensiblement dans un plan ou présenter une convexité pour suivre la courbure de l'os crânien du patient. La structure support 100 est destinée à être fixée dans l'os crânien du patient au niveau de son bord périphérique grâce à des vis d'ancrage ou grâce à tout autre moyen de fixation connu de l'homme du métier.

Dans le mode de réalisation illustré à la figure 1, le dispositif médical 10 comprend neuf transducteurs 101-109 circulaires de 10 millimètres de diamètre chacun. Ces neuf transducteurs 101-109 sont également répartis sur la structure support 100 pour former un réseau de transducteurs, les centres de deux transducteurs adjacents étant espacés d'une distance de 20 millimètres. Ils sont destinés à venir en contact avec la face externe de la dure-mère. Les transducteurs 101-109 sont reliés entre eux par l'intermédiaire d'éléments de connexion électrique 111 - tels que des cartes à circuit imprimé flexo-rigide - pour permettre l'alimentation séquentielle en énergie électrique des transducteurs 101-109 et le transfert de signaux électriques d'activation issus de l'unité de commande externe (non représentée).

La borne de connexion électrique 110 permet de connecter le dispositif médical 10 (une fois celui-ci implanté) à l'unité de commande externe qui alimente les transducteurs 101-109 en énergie électrique, et règle leurs paramètres de fonctionnement. Une telle borne de connexion 110 est notamment connue du document EP 2 539 021 et ne sera pas décrite plus en détails dans la suite. Elle est adaptée pour coopérer avec des moyens de connexion comportant :
- un câble électrique dont l'une des extrémités est reliée à l'unité de commande, et
- une aiguille transdermique raccordée à l'autre extrémité du câble.

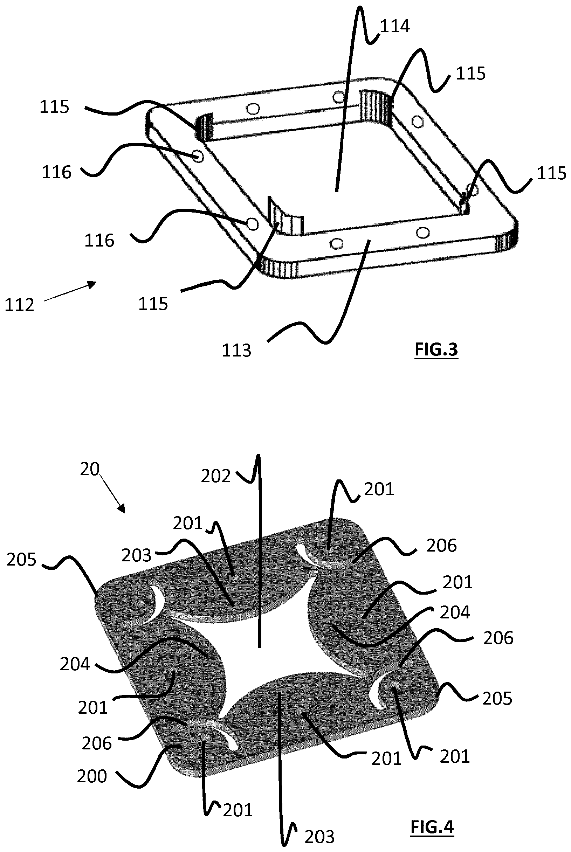

En référence à la figure 3, le dispositif médical 10 peut également comprendre une (ou plusieurs) entretoises 112. Notamment, le dispositif médical 10 peut comprendre deux entretoises 112 de 1 millimètre d'épaisseur et une entretoise 112 de 2 millimètres d'épaisseur. Cette (ou ces) entretoise(s) 112 permet(tent) d'adapter la hauteur de positionnement de la structure support 100 en fonction de l'épaisseur de l'os crânien du patient. Ces entretoises 112 peuvent également être utilisées pour incliner la structure support 100 sur l'os crânien afin d'orienter les transducteurs 101-109 vers le tissu cérébral à traiter, par exemple en découpant une portion d'une entretoise et en l'assemblant à une autre entretoise afin de former une entretoise résultante d'épaisseur variable. Chaque entretoise 112 comprend un cadre périphérique 113 destiné à être positionné entre la structure support 100 et l'os crânien. Les dimensions de l'ouverture centrale 114 de chaque entretoise 112 sont légèrement supérieures aux dimensions de la surface occupée par les transducteurs 101-109 fixés sur la structure support 100 pour permettre le passage de ceux-ci à travers l'ouverture centrale 114.

Avantageusement le cadre périphérique 113 de chaque entretoise 112 comporte des orifices traversants 116 de diamètre supérieur à celui de vis de fixation de l'implant sur l'os afin que le vissage des vis ne vienne pas déformer l'entretoise 112 lors de sa traversée.

De préférence, chaque entretoise 112 comporte également des ergots de positionnement 115 destinés à épouser la forme de l'ouverture crânienne. Ces ergots s'étendent en sailli vers l'extérieur du cadre périphérique 113 ; ils peuvent être disposés dans les angles de l'entretoise (voir figure 3). Ces ergots 115 sont destinés à s'étendre dans l'ouverture réalisée dans l'os crânien du patient lorsque l'entretoise 112 est placée sur le crâne du patient. Ainsi l'entretoise reste centrée dans l'ouverture, sans que le chirurgien ait à la maintenir manuellement lorsqu'il place l'implant.

Le dispositif médical 10 illustré aux figures 1 et 2 est destiné à être inséré dans une ouverture carrée de 58×58 millimètres réalisée par retrait d'un volet osseux de l'os crânien du patient. Pour faciliter le positionnement et le dimensionnement de cette ouverture, le praticien peut utiliser l'accessoire chirurgical 20 illustré à la figure 4.

### 2. Accessoire chirurgical

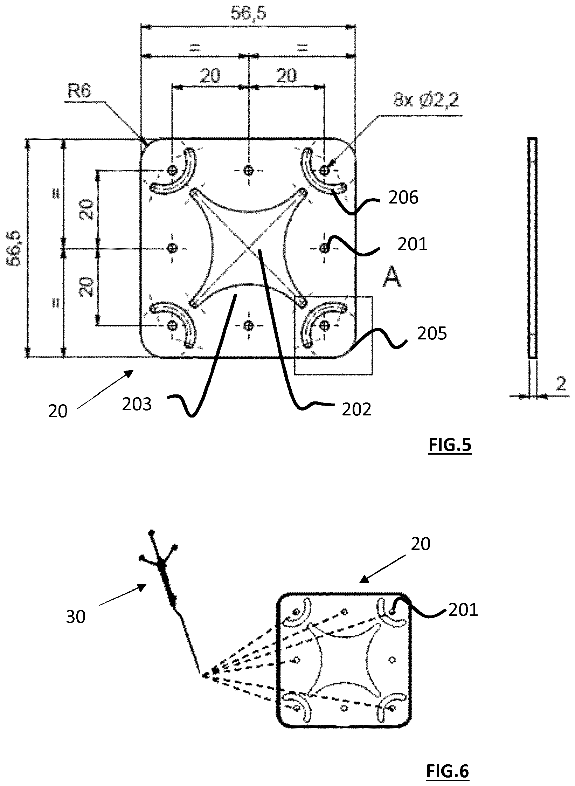

En référence aux figures 4 et 5, l'accessoire chirurgical 20 comprend un corps 200 dimensionné pour se rapprocher des dimensions de la structure support 100 du dispositif médical 10. Le corps 200 est sensiblement plan. Son périmètre externe définit les dimensions de l'ouverture à réaliser dans l'os crânien du patient pour recevoir le dispositif médical 10. Dans le mode de réalisation illustré à la figure 4, le périmètre externe du corps 200 présente une forme sensiblement carrée. De préférence, le corps 200 est réalisé dans un matériau élastiquement déformable, tel que de la silicone de qualité médicale. La (ou les) face(s) du corps 200 peuvent être texturées afin d'assurer de limiter les risques de glissement :
- de l'accessoire chirurgical sur le crâne du patient et/ou
- des doigts du praticien sur l'accessoire chirurgical.

Avantageusement, le corps 200 de l'accessoire chirurgical 20 comprend une pluralité de trous 201. La position de chacun de ces trous 201 coïncide avec la position de l'axe de symétrie d'un transducteur 101-109 respectif sur la structure support 100 du dispositif médical 10. Ainsi, la mise en place de l'accessoire chirurgical sur le crâne du patient permet au praticien de visualiser les positions qu'auront les transducteurs 101-109 une fois le dispositif médical 10 implanté. La présence de ces trous 201 permet au praticien utilisant l'accessoire chirurgical 20 de définir avec précision un positionnement idéal pour l'ouverture à réaliser par rapport à la position du tissu cérébral à traiter. A cet effet, le praticien peut utiliser un pointeur de neuro-navigation 30 connu de l'homme du métier : le positionnement de ce pointeur 30 au niveau de chaque trou 201 lui permet de vérifier (sur des moyens d'affichage) si la position de l'accessoire chirurgical 20 sur le crâne du patient correspond avec une position souhaitée pour le dispositif médical 10 afin d'assurer un traitement efficace du tissu cérébral à traiter (cf. figure 6).

Les trous 201 peuvent être de différentes formes, par exemple circulaire ou oblongue. Dans certaines variantes de réalisation, tous les trous 201 ménagés dans le corps 200 sont de forme identique. Toutefois dans d'autres variantes de réalisation les (ou certains des) trous 201 disposés sur le corps 200 peuvent être de formes différentes. Ceci permet de différentier les différents transducteurs 101-109. La fourniture de cette information supplémentaire au praticien permet de faciliter l'orientation du dispositif médical.

L'accessoire chirurgical 20 comprend également une lumière centrale 202 ménagée au centre du corps 200. Cette lumière centrale 202 permet de faciliter la déformation de l'accessoire chirurgical 20 lors de son placement sur le crâne du patient. On assure ainsi la conformation de l'accessoire chirurgical 20 à la courbure du crâne du patient. La lumière centrale 202 peut présenter différentes formes. Dans le mode de réalisation illustré à la figure 4, la lumière centrale est en forme de X pour augmenter la souplesse du corps 200.

Pour faciliter le placage du corps 200 sur le crâne du patient, l'accessoire chirurgical 20 comprend également une (ou plusieurs) paire(s) de pattes 203, 204 opposées s'étendant vers l'intérieur de la lumière centrale 202. En appliquant ses doigts sur les pattes, le praticien peut maintenir l'accessoire chirurgical 20 en position lors des phases de détermination d'une position optimale pour l'ouverture et de traçage de celle-ci sur le crâne du patient. Dans le mode de réalisation illustré à la figure 4, l'accessoire chirurgical 20 comprend deux paires 203, 204 de pattes opposées, chaque patte s'étendant vers l'intérieur de la lumière centrale.

Les coins 205 du corps 200 peuvent être arrondis. Dans ce cas, le rayon de courbure de l'arrondi de chaque coin 205 est choisi sensiblement égal à la moitié du diamètre d'un foret médical de perçage utilisé durant la réalisation de l'ouverture dans l'os crânien du patient. Ainsi suite au traçage, le praticien dispose d'un repère sur le crâne du patient permettant de faciliter le positionnement du foret médical de perçage.

Pour faciliter encore le positionnement du foret médical de perçage, l'accessoire chirurgical 20 peut comprendre quatre encoches 206 en forme d'arc de cercle disposées en regard d'un coin 205 respectif du corps 200. Là encore, le rayon de courbure des encoches 206 est choisi sensiblement égal à la moitié du diamètre d'un foret médical de perçage. Chaque encoche 206 ainsi formée dont la concavité est orientée vers son coin associé, le centre des encoches 206, correspondant au centre des coins 205, constitue un repère de positionnement du foret médical de perçage du trou de trépan.

### 3. Principe de fonctionnement

Le principe de fonctionnement du kit chirurgical décrit ci-dessus est le suivant.

Le praticien réalise une incision dans le cuir chevelu du patient. La peau et les muscles sont soulevés afin d'exposer le crâne.

Une fois le crâne du patient mis à nu, le praticien plaque l'accessoire chirurgical 20 sur le crâne du patient dans une zone préalablement définie pour recevoir le dispositif médical. Pour ce faire, le praticien positionne ses doigts au niveau des paires de pattes 203, 204 et applique une force tendant à plaquer le corps de l'accessoire contre le crâne du patient.

Le praticien insère le pointeur de neuro-navigation 30 dans les trous 201 de l'accessoire 20. La position et l'orientation du pointeur de neuro-navigation (mesurées par un système de neuro-navigation connu de l'homme du métier) sont reportées dans une image tridimensionnelle préalablement acquise (par exemple grâce à une technique d'imagerie par résonnance magnétique) illustrant le crâne et le tissu cérébral à traiter.

L'image résultante ainsi obtenue est affichée sur des moyens d'affichage pour permettre au praticien de vérifier la position et l'orientation de l'accessoire chirurgical 20.

Une fois l'accessoire chirurgical 20 disposé dans une position et orientation souhaitée, le praticien dessine - en utilisant un stylo médical - le contour du corps 200 sur le crâne du patient. Il trace également sur le crâne du patient les encoches 206.

Le praticien retire ensuite l'accessoire chirurgical 20 pour procéder à la découpe d'un volet osseux.

Le praticien positionne successivement le foret médical de perçage au niveau des différentes zones délimitées par les coins arrondis 205 et les encoches 206. Des trous sont forés dans le crâne (par exemple, 2, 3 ou 4 trous en fonction des besoins du praticien).

Le praticien utilise ensuite une scie chirurgicale (craniotome) pour découper le crâne au niveau des segments rectilignes dessinés sur celui-ci et correspondant au contour de l'ouverture.

Une fois les découpes réalisées, le volet osseux est extrait du crâne : on obtient ainsi l'ouverture dans laquelle le dispositif médical doit être positionné.

Le praticien installe le dispositif médical 10 dans l'ouverture et le fixe sur le contour de l'ouverture.

Une fois le dispositif médical fixé, le praticien remet le cuir chevelu et les muscles en place pour recouvrir le dispositif médical 10.

Le lecteur aura compris que de nombreuses modifications peuvent être apportées à l'invention décrite précédemment sans sortir matériellement des nouveaux enseignements et des avantages décrits ici.

Par conséquent, toutes les modifications de ce type sont destinées à être incorporées à l'intérieur de la portée des revendications jointes.

## Revendications

1. Kit chirurgical à utiliser lors d'une procédure de craniectomie, le kit chirurgical comprenant :
- un dispositif médical (10) à implanter dans un os crânien d'un patient, le dispositif médical (10) incluant une structure support (100) et plusieurs transducteurs (101-109) montés sur la structure support (100) pour l'émission d'ondes ultrasonores de traitement d'un tissu cérébral,
**caractérisé en ce que** le kit chirurgical comprend
- un accessoire chirurgical (20) destiné à être positionné sur l'os crânien du patient, l'accessoire chirurgical (20) incluant un corps (200) dimensionné pour se rapprocher des dimensions de la structure support (100) du dispositif médical (10), le périmètre externe du corps (200) définissant les dimensions d'une ouverture à réaliser dans l'os crânien du patient pour recevoir le dispositif médical (10), l'accessoire chirurgical (20) comprenant une pluralité de trous (201) ménagés dans le corps (200), la position de chaque trou (201) sur le corps (200) coïncidant avec la position de l'axe de symétrie d'un transducteur (101-109) respectif sur la structure support (100), chaque trou définissant un repère pour l'extrémité d'un système de neuronavigation afin de faciliter le positionnement optimal de l'ouverture à réaliser dans l'os crânien.

2. Kit chirurgical selon la revendication 1, *dans lequel* le corps (200) comprend une lumière centrale (202), l'accessoire chirurgical (20) comprenant au moins une paire de pattes opposées (203, 204), notamment deux paires de pattes opposées, chaque patte s'étendant en saillie du corps (200) vers l'intérieur de la lumière centrale (202).

3. Kit chirurgical selon la revendication 2, *dans lequel* la lumière centrale (202) est en forme de X.

4. Kit chirurgical selon l'une quelconque des revendications précédentes, ***dans lequel*** le corps (200) présente une forme sensiblement parallélépipédique à coins arrondis (205), par exemple carrée à coins arrondis, l'arrondi de chaque coin (205) présentant un rayon de courbure sensiblement égal à la moitié du diamètre d'un foret médical de perçage (craniotome) utilisé durant la réalisation de l'ouverture dans l'os crânien du patient.

5. Kit chirurgical selon l'une quelconque des revendications précédentes, ***dans lequel*** le corps (200) présente une forme sensiblement parallélépipédique, par exemple carrée, l'accessoire chirurgical (20) comprenant quatre encoches (206) en forme d'arc de cercle disposées en regard d'un coin (205) respectif du corps (200).

6. Kit chirurgical selon la revendication précédente, *dans lequel* la concavité de chaque encoche (206) est orientée vers son coin associé (205), le rayon de courbure de chaque encoche (206) étant sensiblement égal à la moitié du diamètre d'un foret médical de perçage utilisé durant la réalisation de l'ouverture dans l'os crânien du patient.

7. Kit chirurgical selon l'une des revendications précédentes, *dans lequel* le corps (200) est réalisé dans un matériau déformable ou semi rigide.

8. Kit chirurgical selon la revendication précédente, *dans lequel* le corps (200) est réalisé en silicone de qualité médicale.

9. Kit chirurgical selon l'une quelconque des revendications précédentes, ***dans lequel*** la face inférieure du corps (200) destinée à venir en contact avec l'os crânien du patient et/ou la face supérieure opposée à la face inférieure est texturée.

10. Kit chirurgical selon l'une quelconque des revendications précédentes, lequel comprend en outre au moins une entretoise (112) apte à être positionnée entre le dispositif médical (10) et l'os crânien du patient.

11. Kit chirurgical selon la revendication 10, dans lequel chaque entretoise (112) comprend un cadre périphérique (113) incluant des orifices traversants (116) pour le passage de vis de fixation du dispositif médical sur l'os crânien du patient.

12. Kit chirurgical selon l'une quelconque des revendications 10 ou 11, dans lequel chaque entretoise comprend en outre des ergots (115) s'étendant en saillie vers l'extérieur du cadre périphérique (113).

## Patentansprüche

1. Chirurgisches Kit zur Verwendung bei einem Kraniektomieverfahren, wobei das chirurgische Kit umfasst:
- eine in einen Schädelknochen eines Patienten zu implantierende medizinische Vorrichtung (10), wobei die medizinische Vorrichtung (10) eine Tragstruktur (100) und mehrere Wandler (101 bis 109) umfasst, die auf der Tragstruktur (100) zum Senden von Ultraschallwellen zur Behandlung eines Hirngewebes montiert sind, **dadurch gekennzeichnet, dass** das chirurgische Kit umfasst:
- ein chirurgisches Zubehör (20), das dazu bestimmt ist, an dem Schädelknochen des Patienten positioniert zu werden, wobei das chirurgische Zubehör (20) einen Körper (200) umfasst, der bemessen ist, um sich den Abmessungen der Tragstruktur (100) der medizinischen Vorrichtung (10) anzunähern, wobei der Außenumfang des Körpers (200) die Abmessungen einer Öffnung definiert, die in dem Schädelknochen des Patienten herzustellen ist, um die medizinische Vorrichtung (10) aufzunehmen, wobei das chirurgische Zubehör (20) eine Vielzahl von Löchern (201) umfasst, die in dem Körper (200) eingerichtet sind, wobei die Position von jedem Loch (201) auf dem Körper (200) mit der Position der Symmetrieachse eines entsprechenden Wandlers (101 bis 109) auf der Tragstruktur (100) übereinstimmt, wobei jedes Loch eine Markierung für das Ende eines Neuronavigationssystems definiert, um die optimale Positionierung der in dem Schädelknochen herzustellenden Öffnung zu erleichtern.

2. Chirurgisches Kit nach Anspruch 1, wobei der Körper (200) einen zentralen Schlitz (202) umfasst, wobei das chirurgische Zubehör (20) mindestens ein Paar entgegengesetzter Laschen (203, 204), insbesondere zwei Paar entgegengesetzter Laschen, umfasst, wobei jede Lasche sich von dem Körper (200) hin zum Inneren des zentralen Schlitzes (202) hervorstehend erstreckt.

3. Chirurgisches Kit nach Anspruch 2, wobei der zentrale Schlitz (202) X-förmig ist.

4. Chirurgisches Kit nach einem der vorhergehenden Ansprüche, wobei der Körper (200) eine im Wesentlichen parallelepipedische Form mit abgerundeten Ecken (205), zum Beispiel quadratisch mit abgerundeten Ecken, aufweist, wobei die Abrundung von jeder Ecke (205) einen Krümmungsradius aufweist, der im Wesentlichen gleich der Hälfte des Durchmessers eines medizinischen Bohrers (Kraniotom) ist, der während der Herstellung der Öffnung im Schädelknochen des Patienten verwendet wird.

5. Chirurgisches Kit nach einem der vorhergehenden Ansprüche, wobei der Körper (200) eine im Wesentlichen parallelepipedische, zum Beispiel quadratische Form, aufweist, wobei das chirurgische Zubehör (20) vier kreisbogenförmige Nuten (206) umfasst, die einer jeweiligen Ecke (205) des Körpers (200) gegenüberstehend angeordnet sind.

6. Chirurgisches Kit nach dem vorhergehenden Anspruch, wobei die Konkavität jeder Nut (206) hin zu ihrer zugehörigen Ecke (205) ausgerichtet ist, wobei der Krümmungsradius von jeder Nut (206) im Wesentlichen gleich der Hälfte des Durchmessers eines medizinischen Bohrers ist, der während der Herstellung der Öffnung im Schädelknochen des Patienten verwendet wird.

7. Chirurgisches Kit nach einem der vorhergehenden Ansprüche, wobei der Körper (200) in einem verformbaren oder halbstarren Material hergestellt ist.

8. Chirurgisches Kit nach dem vorhergehenden Anspruch, wobei der Körper (200) aus medizinischem Silikon hergestellt ist.

9. Chirurgisches Kit nach einem der vorhergehenden Ansprüche, wobei die untere Seite des Körpers (200), die dazu bestimmt ist, mit dem Schädelknochen des Patienten in Kontakt zu kommen, und/oder die obere Seite, die der unteren Seite entgegengesetzt ist, strukturiert ist.

10. Chirurgisches Kit nach einem der vorhergehenden Ansprüche, das ferner mindestens einen Abstandshalter (112) umfasst, der geeignet ist, zwischen der medizinischen Vorrichtung (10) und dem Schädelknochen des Patienten positioniert zu werden.

11. Chirurgisches Kit nach Anspruch 10, wobei jeder Abstandshalter (112) einen Umfangsrahmen (113) umfasst, der Durchgangsöffnungen (116) für den Durchgang von Befestigungsschrauben der medizinischen Vorrichtung am Schädelknochen des Patienten umfasst.

12. Chirurgisches Kit nach einem der Ansprüche 10 oder 11, wobei jeder Abstandshalter ferner Vorsprünge (115) umfasst, die sich hin zur Außenseite des Umfangsrahmens (113) hervorstehend erstrecken.

## Claims

1. A surgical kit for use in a craniectomy procedure, the surgical kit comprising:
- a medical device (10) to be implanted in a cranial bone of a patient, the medical device (10) including a support structure (100) and several transducers (101-109) mounted on the support structure (100) for emitting ultrasound waves of treatment of brain tissue,
**characterized in that** the surgical kit comprises:
- a surgical accessory (20) intended to be positioned on the cranial bone of the patient, the surgical accessory (20) including a body (200) dimensioned to approximate the dimensions of the support structure (100) of the medical device (10), the outer perimeter of the body (200) defining the dimensions of an opening to be made in the cranial bone of the patient in order to receive the medical device (10),
the surgical accessory (20) comprising a plurality of holes (201) in the body (200), the position of each hole (201) on the body (200) coinciding with the position of the axis of symmetry of a respective transducer (101-109) on the support structure (100), each hole defining a mark for the end of a neuronavigation system in order to facilitate the optimal positioning of the opening to be made in the cranial bone.

2. The surgical kit according to claim 1, ***wherein*** the body (200) comprises a central lumen (202), the surgical accessory (20) comprising at least one pair of opposite tabs (203, 204), in particular two pairs of opposite tabs, each tab protruding from the body (200) inwardly of the central lumen (202) .

3. The surgical kit according to claim 2, ***wherein*** the central lumen (202) is X-shaped.

4. The surgical kit according to any one of the preceding claims, ***wherein*** the body (200) has a substantially parallelepiped shape with rounded corners (205), for example a square shape with rounded corners, the rounded shape of each corner (205) having a radius of curvature substantially equal to half the diameter of a medical drill bit (craniotome) used when making the opening in the cranial bone of the patient.

5. The surgical kit according to any one of the preceding claims, ***wherein*** the body (200) has a substantially parallelepiped shape, for example a square shape, the surgical accessory (20) comprising four notches (206) in the form of a circular arc disposed facing a respective corner (205) of the body (200).

6. The surgical kit according to the preceding claim, ***wherein*** the concavity of each notch (206) is oriented towards its associated corner (205), the radius of curvature of each notch (206) being substantially equal to half the diameter of a medical drill bit used when making the opening in the cranial bone of the patient.

7. The surgical kit according to any of the preceding claims, ***wherein*** the body (200) is made of a deformable or semi-rigid material.

8. The surgical kit according to the preceding claim, ***wherein*** the body (200) is made of medical-grade silicone.

9. The surgical kit according to any one of the preceding claims, ***wherein*** the lower face of the body (200) intended to come into contact with the cranial bone of the patient and/or the upper face opposite to the lower face is textured.

10. The surgical kit according to any one of the preceding claims, which further comprises at least one spacer (112) adapted to be positioned between the medical device (10) and the cranial bone of the patient.

11. The surgical kit according to claim 10, wherein each spacer (112) comprises a peripheral frame (113) including through-orifices (116) for the passage of fixation screws of the medical device to the cranial bone of the patient.

12. The surgical kit according to any one of claims 10 or 11, wherein each spacer further comprises lugs (115) protruding outwardly of the peripheral frame (113).
